# EUROPEAN PATENT APPLICATION

(11) **EP 1 262 185 A1**
(43) Date of publication of application: **04.12.2002**
(21) Application number: 01113138.0
(22) Date of filing: 30.05.2001
(51) Int. Cl.: A61K 31/70, A61K 31/65, A61K 47/22, A61P 31/04, A61P 31/10

(54) **Synergistic antibiotic compositions**

(71) Applicant: New Pharma Research Sweden AB, 223 70 Lund (SE)
(72) Inventor: Shoa'a, Abdul Rahman, 22476 Lund (SE); Hossny, El-Banna, 13 A El-Siada Khadiga street, Giza (EG); Ra'afat, Kittaneh, 11 115 Amman (JO)
(74) Representative: Johansen, Marianne

(57) **Abstract**

Compositions for the prophylactic and/or therapeutic treatment of diseases, particularly in poultry, comprising tylosin, a tetracycline and an acidic agent. Optionally, the compositions further comprise 2-pyrrolidone. The combination enhances the therapeutic effect of the tetracycline. The compositions are stable upon storage in the form in which they are intended to be administered to poultry. The invention concerns also the combined use of tylosin and a tetracycline for the treatment of animal diseases and the conversion of tylosin A to tylosin B by 2-pyrrolidone.

## Description

### Field of the invention

The present invention concerns compositions comprising a combination of antibiotics and an acidic agent, the compositions being intended to be used in human or veterinary medicine, particularly in veterinary medicine.

### Background of the invention

Diseases caused by pathogenic bacterial agents are known to cause heavy losses in the poultry industry due to high mortality and the rapid spreading of the disease to healthy flocks.

Gram-negative and gram-positive bacteria, as well as mycoplasma have been associated with diseases affecting poultry and other farming animals, like cattle, buffaloes, sheep, pigs and goats.

In the field of, for example, poultry farming or industry, extensive use of antibiotics to treat or protect (on a prophylactic basis) the flocks is made. Because treating individual animals with antibiotics is too expensive, these are added to the drinking water of the entire flock.

In this regard, antibiotics belonging to the group of fluoroquinolones, like enrofloxacin and sarafloxacin have been described and used extensively for the treatment of poultry over the last five years.

However, it has been found that these agents have the drawback of inducing the development of fluoroquinolone-resistant pathogens, like for example of the type Campylobacter, which is not desirable, since these pathogens are transferred to humans when they eat undercooked poultry which carry the resistant pathogens.

For this reason, the Food & Drug Administration of the USA has recently decided to ban these antibiotics because of bacterial resistance. This decision is the result of studies having shown that due to the infection by these resistant pathogens, some of the patients, especially children and the elderly, were likely to have prolonged illness and complications that would be life threatening.

Therefore, there is now a strong need for an alternative agent or composition which has an effective antibacterial activity and a limited, or even zero, resistance-inducing effect on the pathogens.

More specifically, there is a strong need for an antibacterial agent active against bacterial diseases affecting avian species, like the chronic respiratory disease, Colibacillosis, Colisepticemia, Pasteurellosis, Salmonellosis, fowl cholera, necrotic enteritis, ulcerative enteritis, avian spirochetosis, infectious coryza, or other diseases due to pathogenic bacteria like Cholesteridia, Listeria, Staphylococcus and Streptococcus.

There is also a need for an agent which is active against bacterial diseases affecting other animal species, like shipping fever, pneumonia, cough and mycoplasmosis.

An agent of interest has been widely used in veterinary medicine, namely tylosin.

Tylosin is an antibiotic belonging to the group of macrolides. It was the first antibiotic developed exclusively for agricultural use, and was originally isolated from a strain of Streptomyces fradiae found in soil from Thailand. It is now one of the world's leading agricultural antibiotics. It is effective mainly against Gram-positive bacteria and is also one of the most effective antibiotics against mycoplasma.

Combined with a feed additive, tylosin is also used to reduce the incidence of liver abscesses caused by Spherophorus necrophorus and Corynebacterium pyogenes in beef cattle, and to improve feed efficiency and control chronic respiratory disease caused by Mycoplasma gallisepticum in chickens.

In liquid form, tylosin is mixed into drinking water for chickens and turkeys to treat chronic respiratory diseases.

Other known uses of tylosin are by intramuscular injection to treat bovine respiratory complex associated with Pasteurella multocida and Corynebacterium pyogenes, foot rot and calf diphteria caused by Fusobacterium necrophorum, and metritis caused by Corynebacterium pyogenes in beef cattle and non-lactating dairy cattle. It is also administered intramusculary to swine to treat swine arthritis caused by Mycoplasma hyosynoviae, swine pneumonia caused by Pasteurella spp, swine erysipelas caused by Erysipelothrix rhusiopathiae, and acute swine dysentery associated with Treponema hyodysenteriae.

Chemically, tylosin is a sixteen-membered lactone ring substituted with various sugar moieties. Tylosin is a mixture of four related compounds, designated tylosin A, B, C, and D, with tylosin A being the major component. When compared to tylosin B, tylosin A contains an additional mycarosyl group. It is generally also accepted that tylosin A is more active than tylosin B.

Tylosin is not soluble in aqueous compositions. It is sold as a powder, for example by the company Elanco Animal Health, USA, under the trade name Tylan®.

EP 0 221 699 teaches that in order to improve the dissolution rate of tylosin tartrate, an acidifying agent must be added to the composition, the acid salts of alkali metals being preferred, like for example disodium hydrogen citrate. Citric acid is also mentioned as a possible solubilisation enhancer.

A combination of tylosin with several antibiotics has also been reported. These antibiotics are beta-lactam antibiotics, like amoxycillin (EP 0 221 699), or aminoglycoside antibiotics like tobramycin, apramycin, nebramycin 5, gentamycin and neomycin (US 4 283 388 and US 4 379 781). According to the aforementioned documents, these combinations are useful against colibacillosis in weanling pigs, mycoplasma in mammalian tissue culture and Pasteurella haemolytica in cattle.

However, as mentioned by the authors of EP 0 221 699, the combination of tylosin (best soluble in acidic media) and amoxycillin (best soluble in alkaline media) in the same composition is impossible, and therefore both antibiotics must be stored in separate containers. The compartments are then separately emptied into the drinking water tank of the animal. The system requires also that the pH of the drinking water is in the range of from 5.2 to 6.8 and preferably 6.3.

Therefore, there is still a need for an aqueous composition comprising tylosin in combination with another wide spectrum antibiotic, which is stable upon storage in a liquid form.

When looking for agents having a wide spectrum antibacterial activity, oxytetracycline, an antibiotic active against bacterial infectious diseases and produced by Streptomyces aureofaciens and Streptomyces rimosus, was found to be of special interest, since this agent is active against a large variety of Gram-positive and Gram-negative bacteria, as well as mycoplasma, rickettsia and anaplasma.

The structure of oxytetracycline (sold for example under the name Terracortril® by the company Pfizer) is derived from the structure of tetracycline by bearing a hydroxyl group at C-5. As all tetracyclines, it inhibits bacterial protein synthesis by reversible binding to the 30S ribosomal subunit of sensitive bacteria.

Oxytetracycline is sold as a powder. Oxytetracycline is not stable in aqueous solutions. Moreover, it is poorly soluble in water.

To improve the solubility at high concentration, it is known to add an excipient like magnesium compounds or polyvinylpyrrolidone.

However, when used alone these compounds must be added in amounts that increase the viscosity of the preparation to an undesirable level.

Other solutions have been sought, like the use of solvents.

However, the use of solvents alone is also not desirable since if the preparation is intended to be injected in an animal, the solvent causes a strong tissue irritation on the site of injection, which can lead to visible abnormalities in the meat at this place, with an accompanying loss of value.

It is also not possible to attain high concentrations of oxytetracycline using solvents alone. It leads to problems of stability.

There is a need however, for compositions with a higher concentration of the antibiotic, firstly to keep the injection volume as small as possible and second since it has been found that higher dosages with more concentrated solutions achieve a longer therapeutic effect.

On the other hand, the use of tetracycline alone as an antibacterial agent has the disadvantage that bacterial resistance to tetracycline often develops after exposure of the bacteria to the antibiotic, by plasmid mediated interference with transport of the drug to the ribosome, increased rate of removal from the ribosome, or decreased effect on the ribosome.

For some specific diseases, oxytetracycline has been combined with several other antibiotics in the same composition.

For example, WO 84/04249 describes the combination of oxytetracycline with procain benzylpenicillin and colistin sulfate, and teaches that the combination shows a synergistic effect in the treatment of necrotic diseases and mucosa problems. The composition is prepared using magnesium-calcium-oxytetracycline chelates mixed in an aqueous 2-pyrrolidone solution.

A combination of tylosin with oxytetracycline for the treatment of Pasteurella was also reported (Ose E. E., Veterinary Medicine / Small Animal Clinician, January 1996, pp. 92-95).

The experiments in this publication were performed in vitro and in vivo on mice on a research level. This work did not however address the problem of the stability of an aqueous composition containing both antibiotics at high concentrations or in storage.

The results of this work were also restricted to a specific animal species (mice) and to a specific pathogen (Pasteurella multocida).

Furthermore, the findings were not very encouraging since the synergistic effect is only of a magnitude of 1 to 3, as is shown by the results of experiment 3 of Table 1 of the publication. A much higher synergistic effect is expected for the efficient use of a combination of these compounds.

In other respects, for the particular bacterial infections to be treated, the active agents are administered in a specific form, generally selected from injection, topical application or ingestion, i.e. in liquid form.

In the case of liquid formulation, the stability of the active compounds in the composition is of utmost importance, since precipitates can interfere with the optimal dosage of the active substances.

The stability of the composition is also important since after preparation, it is often stored for long periods prior to use.

Surprisingly, the inventors of the present invention have found that a composition comprising a macrolide, oxytetracycline and an acidic agent, is effective as a treatment or prophylaxy of chronic respiratory disease in poultry. The macrolide is preferably tylosin, and the acidic agent ascorbic acid or thioglycolic acid.

The inventors of the present invention have also found that the same effect can be obtained when oxytetracycline is replaced in the composition by any other antibiotic belonging to the group of tetracyclines, such as for example doxytetracycline or chlorotetracycline.

Furthermore, the inventors of the present invention have also found that the compositions are effective against other animal bacterial respiratory diseases and are therefore broad-spectrum antibiotic compositions.

The inventors of the present invention have also found that the compositions are effective to control mycoplasma contamination in mammalian tissue culture.

The compositions can also be used against other bacterial diseases affecting farm animals, and also for some diseases affecting honey bees, like foulbrood.

The compositions can also be used against human bacterial infections, like for example acne.

The inventors of the present invention have also found that compositions comprising the active analogue chemical derivatives of tylosin or of tetracyclines are also effective as broad-spectrum antibiotic compositions.

The inventors of the present invention have also found that when combined with 2-pyrrolidone, the efficacy of the treatment with the compositions in accordance with the present invention is improved and a further enhanced effect is observed.

The inventors of the present invention have also found that when adding 2-pyrrolidone, tylosin A was transformed into tylosin B, and it is presumed that the enhanced effect is due to this transformation.

Thus, the main object of the present invention is to provide new compositions which are effective as antibiotic agents, and preferably agents effective as a treatment or prophylaxy of microbial diseases in humans and animals in general, and particularly in poultry, like chronic respiratory diseases.

Another object of the present invention is to provide these compositions as liquid compositions, which are stable upon storage.

A further object of the present invention is to provide compositions effective as a treatment or prophylaxy of bacterial diseases in animals and which can be used in an effective manner without leading to induction of resistant pathogens.

One of the advantages of the present invention is therefore that the composition can be stored in a stable manner in the form in which it will be administered.

Another advantage is that the composition is efficient against the bacterial diseases without leading to induction of resistant pathogens.

Another advantage is that due to the synergistic effect, less active substance will be used in the treatment, which not only has economic advantages, but also may reduce side effects of the active compounds if any.

Another advantage is that due to the synergistic combination of two antibiotics, it is expected to have much less probability of bacterial resistance, the problem which has been encountered with enrofloxacin and sarafloxacin.

Further problems which can be solved by this invention with respect to known prior art compositions will become apparent to the reader of the following description.

### Summary of the invention

The present invention provides a solution to the aforementioned problems.

The aforementioned object is achieved by a composition having the features defined in claim 1 and comprising a macrolide, a tetracycline and an acidic agent, the macrolide being preferably tylosin and the stabilising agent being selected from ascorbic acid and thioglycolic acid.

The aforementioned object is also achieved by a composition further comprising 2-pyrrolidone.

The aforementioned object is also achieved by the use of the compositions for the treatment of bacterial diseases, and particularly animal bacterial diseases, and most particularly those affecting the respiratory tract of poultry.

Preferred embodiments of the invention, including the use of specific macrolides or tetracyclines, the addition of optional ingredients in the composition like for example other antibiotics, preservatives, vitamins or further stabilising agents, or the use of the composition for the treatment of specific bacterial diseases in animal are defined in the dependent claims.

Unless otherwise specified, the percentages of ingredients used in the compositions are defined as weight per volume.

### Description of preferred embodiments

The bacterial diseases that can be treated by the compositions according to the present invention are any bacterial diseases already known which affect humans or animals, and more specifically those that affect poultry. Preferably, the diseases caused by the following pathogenic agents are aimed at being treated: bacterial diseases affecting avian species, like the chronic respiratory disease, Colibacillosis, Colisepticemia, Pasteurellosis, Salmonellosis, fowl cholera, necrotic enteritis, ulcerative enteritis, avian spirochetosis, infectious coryza, or other diseases due to pathogenic bacteria like Cholesteridia, Listeria, Staphylococcus and Streptococcus, diseases affecting other animal species, like the shipping fever, pneumonia, cough and mycoplasmosis, swine diseases and some diseases affecting the honey bee like foulbrood. Human bacterial infections, like for example acne, are also aimed at being treated.

The amount of macrolide in the composition is preferably between 0,1 and 25% weight/volume. Preferably, the composition contains between 1 and 20% weight/volume of macrolide. Most preferably, the composition contains 20% weight/volume of macrolide.

The macrolide is selected preferably from tylosin, spiromycin, oleandomycin, leucomycin and magnamycin. Most preferably, tylosin will be used.

The tetracycline is preferably present in the composition in amounts of between 0,1 and 25% weight/volume. Most preferably, the composition contains 0.8% weight/volume of a tetracycline.

As already mentioned previously, any antibiotic from the group of tetracyclines is suitable, such as for example oxytetracycline, doxytetracycline or chlorotetracycline. Preferably, oxytetracycline will be used.

These basic substances are provided in the form of their salts with mineral, carboxylic or sulfonic acids. Examples of such acids are the hydrochloride, sulfate, phosphate, tartrate, citrate, laurylsulfate, hexanoate and benzenesulfonate acids. For tylosin, the preferred salts forms are the tylosin tartrate salt and the tylosin phosphate salt. For oxytetracycline, the preferred salt form is the oxytetracycline hydrochloride salt.

The purpose of the combination is to obtain a synergistic and/or additive therapeutic or prophylactic effect.

This effect will be improved by the presence of 2-pyrrolidone in the composition. The amount of 2-pyrrolidone in the composition is preferably between 1 and 50% weight/volume. Most preferably, the amount of 2-pyrrolidone in the composition is 15% weight/volume.

The compositions comprising 2-pyrrolidone will be preferred in the case of treatment by injection. The compositions without 2-pyrrolidone will be preferred in the case of treatment of poultry via drinking water.

The presence of the acidic agent allows the composition to be stored in the form in which it will be used, without loss of activity.

The acidic agent is selected from small organic acids such as ascorbic acid and thioglycolic acid. Preferably, ascorbic acid will be used. The amount of acidic agent in the composition is preferably between 0.1 and 5% weight/volume. The amount of ascorbic acid in the composition is preferably 0.25 or 0.5% weight/volume.

The compositions may further comprise an antibiotic selected from the group consisting of aminoglycoside antibiotics. For example, tobramycin, apramycin, nebramycin, gentamicin and neomycin may be used in this regard.

The compositions may also optionally comprise additional agents like vitamins, preservatives, thickeners or any other agent or additive commonly used in veterinary or medical compositions which is for example useful for the stability of the composition or permits the improvement of the formulation for a specific way of administration without altering the antibacterial activity.

For example, known agents or additives may be added in the composition in order to adjust the pH and/or improve the stability of the composition and/or modify its consistency or smell. These agents or additives are, for example, dimethylformamide, Mg oxide, polyvinylpyrrolidone, triethanolamine, propyleneglycol, MgCl₂ 6.H₂o, sodium carbonate, N-methylpyrrolidone, glycerin, hydroxypropylmethylcellulose, Cremophor®, Tween® 80 and/or Aerosil® 200 (colloidal silicon dioxide).

When necessary, the composition is generally complemented to the required final volume by the addition of deionized water.

The composition is preferably in liquid form, in order to be used for injection, topical application or ingestion. Prior to application, the composition will be diluted in order to obtain the correct dosage form for the treatment.

However, the composition can also be prepared in dried form, for example as dried powder to be diluted in drinking water, or as tablet or bolus. In this case, known agents or additives may be added to the composition such as, for example, binders, fillers, disintegrating agents and/or lubricants. Examples of suitable agents or additives are magnesium stearate, sucrose, lactose, starch, sodium starch glycolate, gum arabic, talc, guar gum, gelatin, sodium carboxymethylcellulose, crospovidone, croscarmellose sodium, sodium bicarbonate, sorbitol, sodium laurylsulphate, povidone K25, pectin, kaolin, flavours and/or sweeteners.

The composition, in its liquid or dried form, can also be incorporated in the animal feed, if necessary.

It should be noted that the form in which the compositions are administered depends upon the particular bacterial infection to be treated or upon the animal species. For example, the mode of treatment is preferably by addition of the composition to the drinking water in the case of poultry. Depending on the severity of the infection, injection or oral therapy may be also indicated. Topical application may be used in the case of infections of a topical nature, such as acne.

For systemic treatment, the composition is administered in order that the total amount of antibiotic is given at a daily dose of about 20 mg/kg body weight, continuously in drinking water in the case of poultry and between three and five days in the case of other animals.

The invention will now be described in more detail with reference to the following examples.

### Examples of compositions

The amounts of ingredients are given in % of weight/volume.
The volume is adjusted by addition of deionized water.

### # Example 1:

A composition comprising:

| | |
|---|---|
| Tylosin base | 20 % |
| Oxytetracycline base | 20 % |
| Ascorbic acid | 0.25 % |
| Propyleneglycol | 45 % |
| Dimethylformamide | 20 % |
| Polyvinylpyrrolidone | 5 % |
| Triethanolamine (to pH 8.5) | 2 % |
| Mg oxide | 1.8 % |
| Water q.s.p. | 100 ml |

Manufacturing method of the composition of Example 1.
Mix 20 g of dimethylformamide with 8 ml water, add 1.8 g of Mg oxide and 0.25 g of ascorbic acid. Then, add the oxytetracycline base and mix for 10 minutes. Then, add 5 g of polyvinylpyrrolidone and 45 g of propyleneglycol. Then, add the tylosin base. Then, add 2 g of triethanolamine, stir for 30 minutes and filter successively on microfilters with meshes of 60, 1.2 and 0.22 µm. The final volume is adjusted to 100 ml with water.

### # Example 2:

A composition comprising:

| | |
|---|---|
| Tylosin base | 20 % |
| Oxytetracycline-HCl | 20 % |
| Ascorbic acid | 0.5 % |
| 2-pyrrolidone | 15 % |
| Dimethylformamide | 37 % |
| Sodium carbonate | 2.13 % |
| Triethanolamine (to pH 8.5) | 10 % |
| MgCl₂ 6.H₂O | 8.20 % |
| Water q.s.p. | 100 ml |

Manufacturing method of the composition of Example 2. Mix 10 g of dimethylformamide with 15 ml water, add 15 g of 2-pyrrolidone, 8.2 g of MgCl₂ 6.H₂O and 0.5 g of ascorbic acid. Then, add the oxytetracycline-HCl and mix for 10 minutes. Then, add 2.13 g of sodium carbonate. Then, add the tylosin base and the rest of the dimethylformamide. Then, add the triethanolamine, stir for 30 minutes and filter successively on microfilters with meshes of 60, 1.2 and 0.22 µm. The final volume is adjusted to 100 ml with water.

### # Example 3:

A composition according to the present invention, which is in soluble powder form and can be mixed with water in proper concentration, contains:

| | |
|---|---|
| Tylosin tartrate | 20 g |
| Oxytetracycline-HCl | 20 g |
| Ascorbic acid | 5 g |
| Aerosil® 200 | 0.1 g |
| Sucrose or lactose or any other suitable filler q.s.p. | 100 g |

### # Example 4:

A composition according to the present invention, which is in soluble powder form and can be mixed with water in proper concentration, contains:

| | |
|---|---|
| Tylosin tartrate | 17 g |
| Doxycycline-HCl | 23 g |
| Ascorbic acid | 5 g |
| Aerosil® 200 | 5 g |
| Sucrose or lactose | |
| or any other suitable filler q.s.p. | 100 g |

### Stability of the compositions upon storage (results obtained with composition of Examples 1 and 2)

To assess the stability of the compositions during storage time, the method described below is used.

The method monitors the presence of the active ingredients (tylosin and/or oxytetracycline in the case of the composition of Example 1 or 2) in the composition in their native active form after storage at 60 °C for several periods of time.

Hence, it has been shown that a composition which is stable for five days in an incubator at 60°C, i.e. shows less than 10% of loss of the active ingredients, is stable on shelf at room temperature for one year.

The principle of the methodology is as follows.

The compositions to be assayed are stored at 60°C in an incubator. Samples are collected at different times, from 0 days to 8 days, and analysed for their composition by HPLC (high-pressure liquid chromatography). The elution profile of the samples are compared to elution profiles of standard compositions containing the active ingredients (tylosin and/or oxytetracycline in the case of the composition of Example 1 or 2) in their native active form. The results are quantified by measurement of the area of the different peaks of elution in the different samples and comparison of the results obtained for the different samples with the results obtained with the standard compositions.

The experimental results obtained when assaying the compositions of Examples 1 and 2 of the present invention are shown in Table 1.

The values given in the table represent the percentage of active ingredients (tylosin and oxytetracycline in the case of Example 1, tylosin B in the case of Example 2) present in their native active form in the sample collected at different times from the compositions of either Example 1 or Example 2, stored at 60°C.

**Table 1**

| Days | Composition of Example 1 | Composition of Example 2* |
|---|---|---|
| 0 | 100 | 100 |
| 1 | 98.33 | 100 |
| 2 | 97.83 | 100 |
| 3 | x | 100 |
| 4 | 94.44 | x |
| 5 | x | 100 |
| 6 | 90.25 | x |
| 8 | 86.04 | x |

| | | |
|---|---|---|
| x means no sample assayed | | |
| * In the case of the composition of Example 2, tylosin B is assayed since it is considered as the active ingredient, converted from tylosin A by the presence of 2-pyrrolidone. | | |

As is clear from the results shown in Table 1, the composition according to Example 1 of the present invention is stable upon storage at 60°C for up to 5 days with less than 10% of degradation of the active ingredients tylosin and oxytetracycline, and the composition according to Example 2 of the present invention is stable upon storage at 60°C for up to 5 days with no detectable degradation of the active ingredient tylosin B.

Therefore, the results are surprisingly very satisfactory and show that the compositions of Examples 1 and 2 are very stable, especially considering that a solution stable for 5 days at 60°C means that it will be stable for one year at room temperature.

### In vitro synergistic effect of the composition in accordance with Example 1 of the present invention, shown by the results of measurements of MIC (Minimum Inhibitory Concentration) against different bacterial strains and mycoplasma - Impact on the treatment of bacterial infections in chicken

To assess the synergistic effect of the composition in accordance with Example 1 of the present invention, an in vitro measurement of the MIC was- performed against different bacterial strains and mycoplasma, using the composition of Example 1 combining tylosin and oxytetracycline and comparing the results obtained to the results of the same experiment performed using compositions containing each agent separately.

The results were also compared to the effect obtained with a composition containing enrofloxacin.

This in vitro test was performed using the agar dilution method recommended by the National Committee for Clinical Laboratory Standards (Antimicrobial Susceptibility Test for Bacteria that Grow Aerobically; NCCLS, USA; 1990).

The following compositions were used:

| | |
|---|---|
| OXY + TYL: (potentiated) | composition in accordance with Example 1 |
| OXY: | composition containing only oxytetracycline as active ingredient |
| TYL A / TYL B: | composition containing only either tylosin A or tylosin B as active ingredient |
| ENRO: | composition containing only oxytetracycline as active ingredient |

The results of the experiments are shown in the following tables, grouping respectively the results obtained against Gram(+) bacteria, Gram(-) bacteria and mycoplasma.

The lower the value of concentration to obtain an inhibitory effect, the more efficient the composition is.

**Table 2**

| BACTERIAL STRAIN Gram (-) | MIC (µg/ml) | | | | |
|---|---|---|---|---|---|
| | OXY | TYL A | TYL B | OXY + TYL | ENRO |
| E. coli ATCC 25922 | 2.0 | > 16.0 | > 16.0 | 0.25 | 0.03 |
| E. coli ATCC 8739 | 2.0 | > 16.0 | > 16.0 | 0.12 | 0.01 |
| E. coli ATCC 10536 | 2.0 | > 16.0 | > 16.0 | 0.12 | 0.01 |
| E. coli pathogenic | > 16.0 | > 16.0 | > 16.0 | > 0.2 | 0.03 |
| Klebsiella pneumoniae 10031 | 0.5 | 62.5 | 31.25 | 0.06 | 0.008 |
| Bordetella bronchiseptica | 0.12 | 125 | 125 | 0.03 | 0.12 |
| Salmonella spp | 2.0 | > 125 | > 125 | 0.12 | 0.03 |
| Proteus | > 16.0 | > 125 | > 125 | > 2.0 | 0.06 |

As can be seen from the results of Table 2, the growth inhibitory effect (MIC) on Gram (-) bacterial strains of a composition in accordance with Example 1 of the present invention, i.e. a composition which combines oxytetracycline and tylosin together with an acidic agent, is in any case at least four times, and in general even much more (up to 4000 times) efficient than the effect of a composition containing either tylosin A, or tylosin B or oxytetracycline alone.

From these results, it should also be noted that even if in general enrofloxacin is more efficient for inhibiting the growth of Gram (-) bacterial strains, the effect obtained with a composition in accordance with Example 1 of the present invention was better in one case (Bordetella bronchiseptica).

**Table 3**

| BACTERIAL STRAIN Gram (+) | MIC (µg/ml) | | | | |
|---|---|---|---|---|---|
| | OXY | TYL A | TYL B | OXY + TYL | ENRO |
| Bacillus subtilis ATCC 6633 | 0.5 | 0.25 | 0.12 | 0.008 | 0.12 |
| Bacillus cereus | 0.5 | 0.25 | 0.12 | 0.01 | 0.12 |
| Bacillus pumillus | 0.5 | 0.25 | 0.06 | 0.008 | 0.03 |
| Micrococcus luteus ATTC 9341 | 0.5 | 0.06 | 0.01 | 0.01 | 0.5 |
| Streptococcus faecium | 0.5 | 0.25 | 0.12 | 0.03 | 0.5 |
| Staphilococcus epidermitis 12228 | 4.0 | 1.0 | 0.25 | 0.01 | 0.06 |
| Staphilococcus aureus ATTC 6538 | 0.25 | 1.0 | 0.25 | 0.01 | 0.12 |
| Staphilococcus aureus ATTC 29737 | 0.25 | 1.0 | 0.25 | 0.01 | 0.12 |

As can be seen from the results of Table 3, the growth inhibitory effect(represented by a lower value of MIC) on Gram (+) bacterial strains of a composition in accordance with Example 1 of the present invention, i.e. a composition which combines oxytetracycline and tylosin together with an acidic agent, is in any case at least the same, and in general even much more (up to 400 times) efficient than the effect of a composition containing either tylosin A, or tylosin B or oxytetracycline alone.

The results also show that tylosin B is more efficient than tylosin A to inhibit the growth of Gram (+) bacterial strains.

From these results, it should also be noted that the inhibition of the growth of Gram (+) bacterial strains with a composition in accordance with Example 1 of the present invention is better than with enrofloxacin.

**Table 4**

| | MIC (µg/ml) | | | | |
|---|---|---|---|---|---|
| | OXY | TYL A | TYL B | OXY + TYL | ENRO |
| Mycoplasma gallisepticum S6 | 0.12 | 0.06 | 0.06 | 0.008 | 0.06 |

As can be seen from the results of Table 4, the growth inhibitory effect on mycoplasma of a composition in accordance with Example 1 of the present invention, i.e. a composition which combines oxytetracycline and tylosin together with an acidic agent, is at least 7.5 times better than the effect of a composition containing either tylosin A, or tylosin B or oxytetracycline alone.

From these results, it should also be noted that the inhibition of the growth of mycoplasma with a composition in accordance with Example 1 of the present invention is better than with enrofloxacin.

The impact of these results on the treatment of bacterial infections in farming chickens can be summarised as follows.

Normally, the average span of life of a chicken in a poultry farm is 45 days, and can be divided into three stages of each 15 days.

During the second stage, the chicken suffers mostly from infections due to Gram (+) micro-organisms and Clostridium. Antibiotics belonging to the group of fluoroquinolones are not very active against the infections in this stage, as can be seen from the results shown in Table 3 (a MIC of at least 0.03 µg/ml and up to 0.12 µg/ml is necessary to obtain an inhibitory effect against most Gram (+) bacterial strains). However, a composition which combines oxytetracycline and tylosin together with an acidic agent is much more efficient against these infections (a MIC of 0.008 µg/ml is sufficient to obtain an inhibitory effect against most bacterial strains, and 0,03 µg/ml provides a complete inhibition against all Gram (+) bacterial strains, as can be seen from the results shown in Table 3). Similar results are obtained when oxytetracycline is replaced by doxycycline in the composition.

During the third stage, diseases mostly due to Gram (-) bacterial infections will affect the flocks. From the results shown in Table 2, one could conclude that a treatment with enrofloxacin is more suitable in this stage than a treatment with a composition in accordance with the present invention, since enrofloxacin is more efficient against most of the bacteria involved in these diseases than a composition in accordance with the present invention.

However, in practice, over the whole span of life of the chicken in the poultry farm, a treatment with a composition in accordance with the present invention will be more efficient than a treatment with enrofloxacin, since the treatment with a composition in accordance with the present invention is already effective against the bacterial infections present in the second stage (Gram (+) bacteria), and thus the chicken is stronger to fight the infections due to the Gram (-) bacteria during the third stage.

### Evaluation of the efficacy of the composition of Example 1 in the treatment of microbial diseases in poultry

In vivo studies were made to evaluate the impact of the composition of Example 1 on the treatment of microbial diseases in poultry.

The microbial diseases concerned by the experiment were diseases caused by infection with either both a pathogenic strain of Escherichia coli and a strain of Mycoplasma gallisepticum or a strain of Mycoplasma gallisepticum alone.

The aim of these studies was to evaluate the effect of the composition on the clinical signs of the diseases and to compare the effect with the effect of a treatment with either enrofloxacin alone or oxytetracycline alone.

The aim of these studies was also to evaluate the effect of a treatment performed via the drinking water and via intra-muscular injection.

Preliminary experiments had shown that an efficient treatment is obtained via drinking water when the composition of Example 1 is given continuously for four days at a dose of 2 ml per litre of drinking water (equivalent to 0.8 g of active agents oxytetracycline and tylosin / litre of drinking water).

Similarly, preliminary experiments had shown that an efficient treatment is obtained via intra-muscular injection when the composition of Example 1 is injected once a day and for three consecutive days at a dose of 10 mg of active agents per kg of body weight.

Seven groups of one day old chickens were used for this experiment. Six groups were infected with Mycoplasma gallisepticum at the second day of age. Five out of these six groups were further infected by a pathogenic strain of Escherichia coli at the 17^{th} day of age. One week after, the birds were treated according to the following Table 5.

**Table 5**

| Group No. | Infection | Treatment | Dose |
|---|---|---|---|
| 1 | No | No treatment | 0 |
| 2 | Mycoplasma & E.coli | No treatment | 0 |
| 3 | Mycoplasma & E. coli | Enrofloxacin | 0.05 g/l via drinking water |
| 4 | Mycoplasma & E.coli | Oxytetracycline | 1 g/l via drinking water |
| 5 | Mycoplasma & E.coli | Composition of Example 1 | 2 ml/l via drinking water |
| 6 | Mycoplasma | Composition of Example 1 | 2 ml/l via drinking water |
| 7 | Mycoplasma & E.coli | Composition of Example 1 | 10 mg/kg body weight via injection |

The effect of the treatment was determined by comparing, in each of the groups, the accumulated mortality (represented by the number of dead birds /total number of birds of the group), the presence of lesions and the presence of the pathogenic microbial strains of Escherichia coli. The evaluation of the presence of lesions and of the presence of the pathogenic microbial strains of Escherichia coli was carried out five days and seven days after stopping of the medication.

The symptoms of the disease caused by the infection by Mycoplasma gallisepticum and Escherichia coli, typical of a chronic respiratory disease, appeared at the fifth day after Escherichia coli infection in the form of difficult breathing, riles, sneezing and nasal discharge.

The post-mortem findings of the diseases were evaluated by examination of the heart, the liver and the lungs for the presence of post-mortem lesions. When present, these lesions were respectively pericarditis, perihepatitis and air saculitis with exudative accumulation.

The results of the experiment are shown in the following Table 6.

**Table 6**

| Group No. | Mortality | Presence of lesions | Presence of pathogenic micro-organisms |
|---|---|---|---|
| 1 | 0/20 | No | No |
| 2 | 7/20 | Yes | Yes |
| 3 | 0/20 | No | No |
| 4 | 5/20 | Yes | Yes |
| 5 | 1/20 | No | No |
| 6 | 0/20 | No | No |
| 7 | 0/20 | No | No |

These results clearly show that the anti-microbial treatment of poultry via oral administration or intra-muscular injection using the composition of Example 1 (results obtained for groups 5, 6 and 7) gives better results than the treatment via oral administration using a composition which contains oxytetracycline alone (results obtained for group 4) and is as good as the treatment via oral administration using a composition which contains enrofloxacin alone (results obtained for groups 3).

Thus, the mortality in the groups treated with the composition in accordance with the present invention (groups 5, 6 and 7) is much lower (1/20 in one of the groups, 0/20 in the others) than the mortality in the group treated with oxytetracycline alone (group 4; 5/20) and comparable to the mortality in the group treated with enrofloxacin alone.

Furthermore, no lesions or pathogenic micro-organisms could be detected in the groups treated with the composition in accordance with the present invention (groups 5, 6 and 7), whereas the presence of lesions and pathogenic micro-organisms could be detected in the groups treated with oxytetracycline only (group 4).

Lastly, from these results it can also be concluded that a composition in accordance with Example 1 of the present invention can be used to treat microbial diseases either via oral administration (groups 5 and 6) or via intra-muscular injection (group 7).

In other respects, the same results as with the composition in accordance with Example 1 of the present invention (results obtained for group 6) were obtained with the composition in accordance with Example 2 of the present invention, when applying the same experimental conditions as for group 6 above, i.e. when treating poultry infected by a strain of Mycoplasma gallisepticum via drinking water at a dose of 1 ml of composition of Example 2 / litre of drinking water for four consecutive days.

### Evaluation of the efficacy of the composition of Example 3 in the treatment of microbial diseases in poultry

In vivo studies were made to evaluate the impact of the compositions in accordance with the present invention on the treatment of microbial diseases in poultry.

The aim of these studies was to evaluate the effect of the composition on the clinical signs of the disease and to compare the effect with the effect of a treatment with each individual antibiotic.

A total number of 400 one day old chickens were purchased. These were infected with Mycoplasma gallisepticum at the second day of age, followed by a pathogenic strain of Escherichia coli at the third day of age. Then, the birds were separated into 6 groups and treated according to the following Table 7.

**Table 7**

| Group No. | Treatment | No. of birds | Dose (mg/l) |
|---|---|---|---|
| 1 | No treatment | 40 | 0 |
| 2 2 | T (10 mg/l) O (10 mg/l) | 200 | 20 mg/l |
| 3 | T (250 mg/l) O (10 mg/l) | 40 | 260 mg/l |
| 4 | T (200 mg/l) | 40 | 200 mg/l |
| 5 | T (500 mg/l) | 40 | 500 mg/l |
| 6 | O (200 mg/l) | 40 | 200 mg/l |

### T refers for tylosin tartrate, O for oxytetracycline-HCl

The treatment was made as follows.

A composition according to Example 3 was prepared, containing the active ingredients tylosin tartrate and oxytetracycline-HCl in proportions as specified in Table 7.

The compositions were administered orally, by dilution in the drinking water, at a dose corresponding to the dose specified in each case in Table 7.

The treatment was started at the day of infection, and continued for five consecutive days.

The effect of the treatment was determined by comparing, in each of the groups, the accumulated mortality (represented by the number of dead birds /total number of birds of the group), the presence of lesions (represented by the number of examined organs -liver, lungs or heart- showing lesions/total number of examined organs isolated from the slaughtered birds) and the presence of the pathogenic microbial strain of Escherichia coli (represented by the number of examined organs -liver, lungs or heart- from which the pathogenic bacteria could be re-isolated /total number of examined organs).

The symptoms of the disease caused by the infection, typical of a chronic respiratory disease, appeared at the second day after Escherichia coli infection in the form of ruffled feather. The post-mortem lesions of the disease were pericarditis, perihepatitis and air saculitis.

The results of the experiment are shown in the following Table 8.

**Table 8**

| Group No. | Mortality | Lesions | Presence of pathogenic micro-organisms |
|---|---|---|---|
| 1 | 7/40 (17.5%) | 26/48 examined organs (54.2%) | 30/48 examined organs (62.5%) |
| 2 | 7/200 (3.5%) | 2/120 examined organs (1.2%) | 2/120 examined organs (1.2%) |
| 3 | 3/40 (7.5%) | 0/48 examined organs (0%) | 0/48 examined organs (0%) |
| 4 | 4/40 (10%) | 8/48 examined organs (16.6%) | 16/48 examined organs (33.3%) |
| 5 | 3/20 (15%) | 9/24 examined organs (37.5%) | 15/24 examined organs (62.5%) |
| 6 | 3/40 (7.5%) | 6/48 examined organs (12.5%) | 16/48 examined organs (33.3%) |

Furthermore, it was observed that the mortality stopped after the second day after medication in group No. 2 and after the first day after medication in group No. 3.

These results clearly show that the composition in accordance with the present invention (results obtained for groups 2 and 3), by combining the effect of tylosin and oxytetracycline, is more suitable for the treatment of chronic respiratory disease in poultry than a composition which contains either tylosin or oxytetracycline (results obtained for groups 4, 5 or 6).

Thus, the mortality in the groups treated with the composition in accordance with the present invention (groups 2 and 3) is at least the same or lower than the mortality in the groups treated with either tylosin or oxytetracycline.

Furthermore, the presence of lesions in the groups treated with the composition in accordance with the present invention (groups 2 and 3) is much lower (0% or 1.2%) than the presence of lesions in the groups treated with either tylosin or oxytetracycline (at least 12.5%).

Lastly, the presence of pathogenic micro-organisms in the groups treated with the composition in accordance with the present invention (groups 2 and 3) is also much lower (0% or 1.2%) than the presence of pathogenic micro-organisms in the groups treated with either tylosin or oxytetracycline (at least 33.3%).

From these results, it can also be concluded that a composition in accordance with the present invention and comprising as low as 10 mg of tylosin and as low as 10 mg of oxytetracycline is already more suitable for the treatment of chronic respiratory disease in poultry than compositions comprising either 200 mg of tylosin or 200 mg of oxytetracycline.

### Dose titration and clinical studies of the efficacy of the composition of Example 1 in the treatment of calves naturally infected with sensitive bacterial strains

In vivo dose titration studies were conducted on calves naturally infected with sensitive bacterial strains in order to determine the efficacy of four different administration doses and to select a low dose end for controlling the disease.

Then, the efficacy of the selected dose was confirmed in clinical field studies conducted on calves naturally infected with sensitive bacterial strains.

In all studies, the route of administration of the composition was by deep intra-muscular injection.

For the dose titration study, 25 calves aged 1 to 4 months were classified in 5 groups of each 5 animals. All the animals showed severe respiratory manifestations including cough, rale and increased rectal body temperature (between 39.5 and 41°C). The diagnosis was pneumonia associated with cough and a rise of the body temperature.

One group of animals was kept untreated, and the other groups were each treated with a different dose of a composition in accordance with Example 1, as shown in Table 9.

The administration doses tested in the study were 0.25, 0.5, 1 and 2 ml /20 kg body weight of a composition in accordance with Example 1, injected as a single dose or as several doses spaced by 48 hours, as shown in Table 9.

The curing and improvement times, the change of the body temperature, as well as the mortality and the presence or absence of an irritation at the site of injection were evaluated in each case over ten days after medication. The results are shown in Table 9.

**Table 9**

| Group No. (5 animals /group) | 1 | 2 | 3 | 4 | 5 |
|---|---|---|---|---|---|
| Treatment (ml /20 kg body weight) and number of doses | -- | 0.25 3 doses | 0.5 3 doses | 1.0 1 dose (4 animals) 2 doses (1 animal) | 2.0 1 dose (4 animals) 2 doses (1 animal) |
| Body temperature | > 40.5°C | > 40°C | > 41°C, but returned to normal in 4 calves 3 days after last dose | > 41.5°C, but returned to normal in 5 calves 2 days after last dose | > 41.5°C, but returned to normal in 5 calves 2 days after last dose |
| Time of curing curing | -- | -- | 1 animal cured after 2^{nd} dose | 4 animals cured after 1^{st} dose | 4 animals cured after 1^{st} dose |
| Time of improvement | -- | 3 animals improved 5 days after last dose | 3 animals improved after 3^{rd} dose | 1 animal improved after 2^{nd} dose | 1 animal improved after 2^{nd} dose |
| Presence of irritation (injection site) | -- | Slight, after 3^{rd} dose | Slight, after 3^{rd} dose | -- | Slight |
| Mortality | 3 animals | 2 animals | -- | -- | -- |

These results clearly show that the efficacy of the treatment increases when the dose is increased from 0.25 to 1 ml /20 kg body weight and that it is stabilised above 1 ml /20 kg body weight.

Thus, when compared to untreated animals (results for group 1), the treatment with 0,25 ml /20 kg body weight (results for group 2) reduced the mortality from 3 to 2 animals and improved the health of the 3 remaining animals 5 days after the last dose was administered.

The treatment with 0,5 ml /20 kg body weight (results for group 3) reduced the mortality to 0, improved the health of 3 animals after the 3^{rd} dose was administered, permitted to cure an animal after the 2^{nd} dose was administered and restored the body temperature to its normal value in 4 animals.

The treatment with 1 or 2 ml /20 kg body weight (results for groups 4 and 5) further improved the health of 1 animal already after the 2^{nd} dose was administered, permitted to cure 4 animals after the 1^{st} dose was administered and restored the body temperature to its normal value in all animals.

The results also show that no irritation occurs at the site of injection when the treatment is effected with a dose of 1 ml /20 kg body weight (result for group 4), whereas a slight irritation can be detected with 2 ml /20 kg body weight (result for group 5) or when 3 doses are given (results for groups 2 and 3).

Therefore, it can be concluded that the best results were obtained when using a single or a double administration of the composition of Example 1 at a dose of 1 ml /20 kg body weight, which was then chosen as the low dose end for controlling the disease.

For the clinical study, 20 calves aged 1.5 to 3.5 months were classified in 3 groups, two of which being of 5 animals and one of 10 animals. All the animals showed severe respiratory manifestations including cough, rale and increased rectal body temperature (between 40.5 and 41.5°C). The diagnosis was pneumonia associated with cough and a rise of the body temperature, as well as secondary bacterial (Gram +) infections associated with viral diseases.

One group of 5 animals was kept untreated (group 1). The group with 10 animals (group 2) was treated at a dose of 1 ml /20 kg body weight using a composition in accordance with Example 1 and a single or double (spaced by 48 hours) dose. The last group of 5 animals (group 3) was treated with enrofloxacin at a dose of 5 mg /kg body weight daily for four consecutive days, for comparison.

The curing and improvement times, the change of the body temperature, as well as the mortality and the presence or absence of the pathogenic bacteria (isolated from nasal discharge) were evaluated in each case over ten days after medication. The results are shown in Table 10.

**Table 10**

| Group No. | 1 2 (5 animals) | (10 animals) | 3 (5 animals) |
|---|---|---|---|
| Treatment and number of doses | -- | Composition of example 1 | Enrofloxacin |
| | -- | 1 dose (8 animals) | 4 doses |
| | | 2 doses (2 animals) | |
| Body temperature | > 40.5°C | > 41.5°C, but returned to normal in 9 calves 2 days after last dose | > 41.5°C, but returned to normal in 3 calves 2 days after last dose |
| Time of curing | -- | 8 animals cured after 1^{st} dose | 3 animals cured after 4^{th} dose |
| Time of improvement | -- | 1 animal improved after 2^{nd} dose 1 animal improved after additional 3^{rd} dose | 1 animal improved after additional 5^{th} dose |
| Presence of pathogenic bacteria | Yes | No | Yes, in 2 animals |
| Mortality | 3 animals | -- | 1 animal |

These results clearly show the higher efficacy of the treatment of the calves with a composition in accordance with Example 1 at a dose of 1 ml /20 kg body weight, when compared to a treatment with enrofloxacin.

Thus, when compared to untreated animals (results for group 1), the treatment with enrofloxacin (results for group 3) reduced the mortality from 3 to 1 animal, permitted to cure 3 animals out of 5 after the 4^{th} dose was administered, improved the health of the 5^{th} animal after an additional 5^{th} dose was administered, and restored the body temperature to its normal value in 3 animals out of 5.

The treatment with a composition in accordance with Example 1 (results for group 2) reduced the mortality to 0, permitted to cure 8 animals out of 10 after the 1^{st} dose was administered, improved the health of 1 further animal after the 2^{nd} dose was administered and of the 10^{th} animal after an additional 3^{rd} dose was administered, and restored the body temperature to its normal value in 9 animals out of 10.

Therefore, it can be concluded that better results were obtained when performing the treatment with a composition of Example 1 when compared to a treatment with enrofloxacin.

Furthermore, the results show that a composition in accordance with Example 1 is suitable for the treatment via intra-muscular injection at a dose of 1 ml /20 kg body weight of calves naturally infected with sensitive bacterial strains and showing symptoms of pneumonia associated with cough and a rise of the body temperature, as well as secondary bacterial (Gram +) infections associated with viral diseases.

### Industrial applicability: treatment of microbial diseases

The compositions according to the present invention can be used to treat animals infected by the aforementioned microbial diseases.

For example, the compositions of examples 1, 2 or 3 can be used to treat poultry infected with Mycoplasma gallisepticum and pathogenic strains of Escherichia coli, which infections lead to a chronic respiratory disease.

The compositions in accordance with the present invention can also be used for the treatment of Colibacillosis, Salmonellosis, Pasteurellosis in poultry.

The compositions in accordance with the present invention have been successfully used for the treatment of chronic respiratory and necrotic enteritis diseases in poultry, of infectious synovitis disease in turkeys and of respiratory microbial diseases in calves, sheeps and goats (natural infections, pneumonia, mastitis as well as secondary bacterial infections associated with viral diseases) on a large number of animals.

In this regard, the compositions comprising 2-pyrrolidone have been particularly efficient for the treatment of respiratory microbial diseases in calves, buffaloes, sheep, pigs and goats.

The compositions in accordance with the present invention can also be used for the treatment of swine diseases.

Although the present invention has been described with reference to several examples and embodiments of specific compositions and concentrations of ingredients, this is not to be considered as a limitation of the invention but merely illustrative thereof.

Specifically, other compounds like chemical derivatives of the active compounds cited herein could be used, as soon as the modification does not lead to a substantial loss of the activity of the compound.

## Claims

1. A composition comprising:
(i) a macrolide;
(ii) a tetracycline;
(iii) an acidic agent selected from ascorbic acid or thioglycolic acid.

2. A composition according to claim 1 wherein the macrolide is tylosin.

3. A composition according to claim 1 or 2 wherein the tetracycline is oxytetracycline.

4. A composition according to any one of claim 1 to 3 wherein the stabilising agent is ascorbic acid.

5. A composition according to any one of claims 1 to 4 wherein the amount of acidic agent is between 0.1% and 5% weight/volume.

6. A composition according to claim 5 wherein the amount of acidic agent is 0.25 or 0.5% weight/volume.

7. A composition according to any one of claims 1 to 6 wherein the amount of macrolide is between 0.1% and 25% weight/volume.

8. A composition according to claim 7 wherein the amount of macrolide is between 1% and 20% weight/volume.

9. A composition according to claim 8 wherein the amount of macrolide is 20% weight/volume.

10. A composition according to any one of claims 1 to 9 wherein the amount of tetracycline is between 0.1% and 25% weight/volume.

11. A composition according to claim 10 wherein the amount of tetracycline is 0.8% weight/volume.

12. A composition according to any one of claims 1 to 11 wherein the composition further comprises 2-pyrrolidone.

13. A composition according to claim 12 wherein the amount of 2-pyrrolidone is between 1% and 50% weight/volume.

14. A composition according to claim 13 wherein the amount of 2-pyrrolidone is 15% weight/volume.

15. A composition according to any one of claims 12 to 14 wherein tylosin is in the form of tylosin B.

16. A composition according to any one of claims 1 to 15 which further comprises other agents or additives commonly used in veterinary or medical compositions like vitamins, preservatives, pH adjusting agents, thickeners or ingredients necessary to permit an oral administration or an administration by way of spray, injection or topical application, or in the feed or drinking water.

17. A composition according to claim 16 wherein the agents or additives are selected from dimethylformamide, Mg oxide, polyvinylpyrrolidone, triethanolamine, propyleneglycol, MgCl₂ 6.H₂o and sodium carbonate.

18. A composition according to any one of claims 1 to 17 which further comprises an antibiotic selected from the group of aminoglycoside antibiotics, such as tobramycin, apramycin, nebramycin, gentamicin or neomycin.

19. A composition according to any one of claims 1 to 18 for use as a medicament.

20. Use of a composition according to any one of claims 1 to 18 for the preparation of a medicament for the prophylactic or therapeutic treatment of human or animal diseases caused by microbial infection.

21. Use of a composition according to any one of claims 1 to 18 to prevent bacterial contamination and contamination by mycoplasma in mammalian tissue culture.
